# FASCICULE DE BREVET EUROPEEN

(11) **EP 0 539 277 B1**
(45) Date de publication et mention de la délivrance du brevet: **10.01.1996**
(21) Numéro de dépôt: 92402853.3
(22) Date de dépôt: 15.10.1992
(51) Int. Cl.: B01J 27/053, B01J 27/02, C07C 2/62

(54) **Utilisation d'un catalyseur en alkylation de paraffines**
Verwendung von einem Katalysator zur Alkylierung von Paraffinen
Use of a catalyst for paraffins alkylation

(30) Priorité: 25.10.1991 FR 9113303; 28.02.1992 FR 9202482
(43) Date de publication de la demande: 28.04.1993
(73) Titulaire: INSTITUT FRANCAIS DU PETROLE, F-92502 Rueil-Malmaison (FR)
(72) Inventeur: Joly, Jean-François, F-75003 Paris (FR); Benazzi, Eric, F-78170 La Celle Saint Cloud (FR); Marcilly, Christian, F-78800 Houilles (FR)

(56) Documents cités:
- EP-A- 0 047 540
- EP-A- 0 259 105
- EP-A- 0 303 005
- US-A- 4 008 178
- US-A- 4 148 758

## Description

La présente invention concerne l'utilisation d'un catalyseur à base de silice et d'acide sulfurique en alkylation catalytique d'isobutane et/ou d'isopentane au moyen d'au moins une oléfine, qui permet d'obtenir au moins un produit par exemple dans le groupe constitué par les diméthylbutanes, les triméthylpentanes, les triméthylhexanes et les triméthylheptanes.

On sait que, pour alimenter les moteurs à combustion interne et à allumage commandé, et notamment les moteurs à taux de compression élevé, il est particulièrement intéressant de disposer de carburants à hauts indices d'octane, c'est-à-dire constitués essentiellement par des hydrocarbures paraffiniques fortement ramifiés. L'alkylation des isoparaffines (isobutane et isopentane) par les oléfines contenant de 3 à 6 atomes de carbone par molécule permet d'obtenir de tels produits. Cette réaction nécessite la mise en oeuvre de catalyseurs très acides, dans le but notamment de réduire les réactions parasites telles que les réactions d'abstraction d'hydrure de l'oléfine et de polymérisation qui fournissent des hydrocarbures peu ramifiés de faible indice d'octane et des hydrocarbures insaturés, les réactions de craquage et les réactions de dismutation.

Les procédés existant pour la production d'hydrocarbures par alkylation de l'isobutane par les oléfines utilisent soit l'acide sulfurique soit l'acide fluorhydrique comme catalyseur. Dans ces procédés le catalyseur acide constitue une phase liquide qui est mise en contact avec le mélange isobutane-oléfine liquide pour former une émulsion. Ces procédés sont coûteux et posent d'importants problèmes vis-à-vis de la sécurité des personnes et vis-à-vis de l'environnement. Afin de remédier à ces problèmes, des systèmes catalytiques différents des acides sulfurique et fluorhydrique en phase liquide ont été recherchés.

Pour catalyser les réactions d'alkylation des isoparaffines par les oléfines, on a déjà proposé de mettre au point des catalyseurs acides à partir de nombreux solides acides de natures différentes. Parmi les familles de catalyseurs acides on peut citer les tamis moléculaires (voir par exemple US-A-3.236.762, US-A-3.251.902, US-A-3.644.565, US-A-4.377.721, US-A-4.384.161 et US-A-4.300.015), les résines macroréticulaires éventuellement associées avec BF₃ (voir par exemple US-A-3.855.342, US-A-3.855.343, US-A-3.862.258 et US-A-3.879.489), les résines perfluorées de type NAFION (voir par exemple US-A-4.056.578 et US-A-4.038.213), les acides de Lewis et/ou de Bronsted déposés sur divers supports inorganiques (voir par exemple US-A-3.975.299, US-A-3.852.371 et US-A-3.979.476), les alumines chlorées (voir par exemple US-A-3.240.840, US-A-3.523.142, US-A-3.607.859, US-A-3.523.142, US-A-4.066.716, US-A-4.083.800 et US-A-4.066.716), les graphites intercalés par des acides de Lewis et/ou de Bronsted (voir par exemple US-A-4.083.885, US-A-4.116.880, US-A-4.128.596 et US-A-3.976.714) et les anions déposés sur supports oxydes tels que ZrO₂/SO₄ (voir par exemple J-01288329, J-01245953 et J-61242641). Ces solides conduisent à la production d'isoparaffines ramifiées mais souffrent de plusieurs défauts majeurs, parmi lesquels on peut citer l'utilisation de rapports molaires isobutane/oléfine souvent très élevés pour limiter l'importance des réactions secondaires et la faible stabilité dans le temps de l'activité catalytique (inhibition du catalyseur par dépôt d'oligomères insaturés) ; ces catalyseurs doivent alors être fréquemment régénérés. De plus, la faible acidité de certains solides acides, tels que les tamis moléculaires par exemple, impose l'utilisation de températures de réaction élevées ce qui est préjudiciable à l'obtention d'hydrocarbures d'indices d'octane élevés.

Dans la présente invention, on a découvert l'utilisation d'un catalyseur permettant d'obtenir des composés paraffiniques à hauts degrés de ramification et hauts indices d'octane par alkylation d'isoparaffine (isobutane et/ou isopentane) par au moins une oléfine comprenant de 3 à 6 atomes de carbone par molécule. Ce catalyseur est avantageusement mis en oeuvre dans un procédé où l'oléfine et/ou un mélange d'oléfines est introduit(e) dans le réacteur en phase liquide et en mélange avec l'isoparaffine et/ou un mélange d'isoparaffines.Le catalyseur est mis en oeuvre en lit fixe, lit mobile ou lit fluide, ou encore en suspension dans la phase liquide des réactifs soumise à une agitation efficace.

Le catalyseur utilisé dans la présente invention renferme de la silice et une phase acide comprenant de l'acide sulfurique, la silice étant imprégnée partiellement ou totalement par ladite phase acide. La concentration de l'acide sulfurique est avantageusement comprise entre 5 et 100 % en poids, de préférence entre 50 et 100 % en poids et de manière encore plus préférée entre 88 et 100 % en poids.

De nombreuses sources de silice peuvent être utilisées ; la surface spécifique de ladite silice est comprise entre 0,01 et 1500 m²/g, de préférence entre 0,01 et 150 m²/g et de manière souvent plus préférée entre 0,01 et 50 m²/g. Le volume poreux total de ladite silice est compris entre 0,005 et 1,5 cm³/g, de préférence entre 0,005 et 1 cm³/g. La silice peut contenir des impuretés comme par exemple les oxydes, les alcalins, les alcalino-terreux, les composés d'aluminium ou toute autre impureté connue de l'homme du métier, la quantité totale de ces impuretés n'excédant pas 5 % en poids, de préférence 2 % en poids par rapport à la silice.

Lors de l'imprégnation de ladite silice, la phase acide comprenant la solution d'acide H₂SO₄ occupe une fraction du volume poreux total comprise entre 5 et 100 %. Le catalyseur ainsi obtenu est caractérisé par une surface spécifique comprise entre 0,01 et 500 m²/g, de préférence entre 0,01 et 150 cm²/g et de manière encore plus préférée entre 0,01 et 40 m²/g.

Il est possible d'ajouter dans la phase acide au moins un additif visant à améliorer les performances catalytiques. L'additif est choisi dans le groupe formé par : H₃PO₄, BO₃H, BF₄H, FSO₃H, CF₃COOH, SbF₅, CF₃SO₃H et SO₃.

Dans ce cas, lors de l'imprégnation de la silice, la phase acide comprenant l'acide sulfurique et au moins un additif occupe une fraction du volume poreux total de la silice comprise entre 5 % et 100 %, et de préférence entre 60 % et 90 % ; le catalyseur ainsi obtenu est caractérisé par une surface spécifique comprise entre 0,01 et 500 m² /g, de préférence entre 0,01 et 150 m²/g et de manière encore plus préférée entre 0,01 et 40 m²/g.

Un additif encore plus préféré selon la présente invention est l'anhydride sulfurique SO₃. Dans le cas de son utilisation, la phase acide comprenant au moins l'acide sulfurique et l'anhydride sulfurique est communément appelé "oléum". La teneur pondérale en anhydride sulfurique dans l'oléum utilisé est comprise entre 0,01 et 60% et de façon préférée entre 1 et 30%. Dans le cas de l'utilisation dudit oléum pour l'imprégnation, il est préféré l'ajout à l'oléum utilisé d'un additif supplémentaire visant à renforcer l'acidité du catalyseur et donc à en améliorer les performances catalytiques. L'additif supplémentaire préféré est l'acide borique (H₃B O₃), la teneur pondérale en acide borique au sein du mélange comprenant l'acide sulfurique et l'anhydride sulfurique est avantageusement comprise entre 0,01 et 50% et de manière encore plus préférée entre 0,01 et 10%.

Le procédé de préparation du catalyseur comprend deux étapes. Dans une première étape la silice est calcinée à une température supérieure à 50 °C, de préférence supérieure à 80 °C et de manière encore plus préférée comprise entre 200 et 600°C, par exemple égale à environ 500 °C. La durée de cette étape de calcination est habituellement comprise entre 10 minutes et 50 heures. La calcination peut être effectuée en présence d'air ou de mélange air/azote, de débit compris entre 0,001 et 10 l/h/g. La seconde étape consiste en l'imprégnation de ladite silice calcinée par la phase acide. Pour réaliser cette étape on peut utiliser toutes les techniques connues de l'homme du métier. Conservé à une température inférieure à la température de fusion de ladite phase acide et conservé à l'abri de l'humidité, le catalyseur selon la présente invention contient ainsi de la silice imprégnée de phase acide solide.

Le catalyseur est utilisé pur ou dilué avec divers matériaux présentant peu d'activité catalytique dans la réaction considérée comme par exemple la silice, l'alumine, la magnésie ou encore diverses argiles telles que par exemple la bentonite, la montmorillonite ou le kaolin.

Le mélange isoparaffine(s)-oléfine(s) est introduit dans le réacteur à une vitesse spatiale horaire, exprimée en poids d'oléfine introduite par unité de poids du catalyseur et par heure comprise entre 0,001 et 10 h⁻¹ et de préférence comprise entre 0,002 et 2 h⁻¹. Ledit mélange peut aussi être réalisé à l'intérieur du réacteur. Dans tous les cas le mélange ainsi constitué est dans le réacteur dans des conditions de pression et de température telles que le mélange d'hydrocarbures reste liquide sur le catalyseur et que les constituants du catalyseur restent à l'état solide.

La température de réaction peut être comprise entre -50 et 150° C, mais nous avons découvert que, d'une façon surprenante, les performances catalytiques sont nettement améliorées lorsque la température de réaction est inférieure à la température de cristallisation de la phase acide utilisée pour l'imprégnation de la silice. La température de réaction doit alors être inférieure à +6 °C, de préférence à 0 °C et de manière préférée inférieure à 5° C et de manière encore plus préférée inférieure à - 10° C. La pression du réacteur est suffisante pour maintenir les hydrocarbures à l'état liquide dans le réacteur.

L'un des avantages du catalyseur tel que la phase acide est constituée principalement d'acide sulfurique est la possibilité d'alkyler l'isobutane et/ou l'isopentane à des températures inférieures à -10 °C et pouvant atteindre -30 °C. En effet, L. F. Albright et al. dans Ind. Eng. Chem. Res. **1988**, 27, pp. 381-397 indiquent très clairement l'intérêt qu'il y a à procéder à l'alkylation de l'isobutane en présence d'acide sulfurique à des températures inférieures à 0° C : baisse très importante des réactions secondaires, et donc de la consommation du catalyseur, augmentation de la qualité des hydrocarbures obtenus. Les résultats publiés ne font référence qu'à des essais réalisés à l'échelle du laboratoire. L'inconvénient lié à l'utilisation de telles températures est la nécessité d'une agitation extrêmement puissante étant donnée la très forte viscosité de l'acide sulfurique en solution à ces températures, voire même l'impossibilité d'agiter si la température est inférieure à la température de fusion de l'acide sulfurique. Le catalyseur utilisé dans la présente invention, lui, permet de procéder à l'alkylation de l'isobutane et/ou de l'isopentane à ces très basses températures sans la nécessité d'une augmentation de la puissance de l'agitation, la phase acide sulfurique étant contenue au sein de la porosité de la silice.

Afin de limiter les réactions secondaires, on peut utiliser un excès d'isoparaffine(s) par rapport à l'oléfine. A titre d'exemple, dans le cas de l'alkylation de l'isobutane par un butène, l'isobutane peut être introduit pur dans la charge ou sous la forme d'un mélange de butanes contenant par exemple au moins 40 % d'isobutane. De plus, on peut introduire un butène pur ou encore un mélange de butènes isomères. Dans tous les cas, le rapport molaire isobutane/butènes dans la charge est compris entre 1 et 100, de préférence entre 3 et 50 et de manière souvent préférée entre 5 et 10.

Les produits de la réaction peuvent être contrôlés régulièrement par mesure de l'indice de brome, par exemple selon le projet de Norme Française Pr. M. 07.071 de mars 1969.

Lorsque la nature du catalyseur et les conditions de travail du catalyseur sont judicieusement choisies (en particulier la température), le catalyseur selon l'invention permet la production de produits d'alkylation des paraffines par les oléfines qui sont intéressants comme carburants pour les moteurs et constituants d'essence et qui comprennent par exemple au moins 60 % moles de paraffines possédant 8 atomes de carbone par molécule et moins de 1 % moles de composés non saturés, les paraffines comprenant 8 atomes de carbone par molécule comprenant 70 à 98 % en moles de triméthylpentanes.

Un autre avantage du catalyseur utilisé dans la présente invention est la possibilité d'alkyler, à basse température, l'isobutane avec des mélanges d'oléfines comportant de 3 à 6 atomes de carbone par molécule, où la proportion d'oléfines comportant au moins 5 atomes de carbone par molécule est très importante (au moins 10 % poids, de préférence au moins 40 % poids).

Les exemples suivants illustrent l'invention sans toutefois en limiter la portée.

### Exemple 1 (selon l'invention) CATALYSEUR ACIDE SULFURIQUE SUR SILICE.

### Préparation du catalyseur A

On active 16 g de silice macroporeuse de surface spécifique égale à 27 m²/g et de volume poreux total égal à 0,78 cm³/g, par calcination sous air pendant 4 heures à 500°C. La silice ainsi activée est conservée sous argon. On procède alors à une imprégnation à sec de 14 g de ladite silice par 20 g d'une solution d'acide sulfurique à 96 % en poids. Le solide ainsi obtenu, appelé catalyseur A, contient 20 g d'acide sulfurique et 14 g de silice, et est conservé sous argon à -18 °C.

### Alkylation de l'isobutane par le pentène-1

On introduit 34 g du catalyseur A préparé selon la méthode décrite ci-dessus dans un réacteur en verre du type Fischer & Porter d'un volume de 360 ml préalablement purgé sous débit d'argon. Le réacteur contenant le catalyseur A est alors fermé, mis sous vide primaire, puis il est refroidi à la température de -20 °C.

80 cm³ d'isobutane sont alors ajoutés dans le réacteur contenant le catalyseur sous agitation, ledit réacteur étant immergé dans un bain froid à -20 °C. On laisse sous agitation le système catalyseur A + isobutane pendant une durée de 30 minutes afin d'homogénéiser la température.

On ajoute régulièrement 1,73 cm³ de pentène-1 par heure pendant une durée totale de 12 heures, la température du réacteur est maintenu à -12 °C pendant toute la durée de l'injection.

Après réaction, la phase hydrocarbure est soutirée du réacteur, puis l'isobutane est évaporé lentement et on recueille l'alkylat qui est analysé par chromatographie en phase vapeur. Sa composition pondérale est donnée dans le tableau 1 ci-après. La conversion de l'oléfine est de 100 %.

**Tableau 1**

| | |
|---|---|
| iC₅ | 3,20 |
| C₆ | 2,65 |
| C₇ | 0,88 |
| C₈ | 11,70 |
| C₉ | 75,40 |
| C₉⁺ | 6,17 |

La fraction C₈ contient 90,3 % poids de triméthylpentanes, la fraction C₉ contient 90,8 % de triméthylhexanes.

### Alkylation de l'isobutane par une coupe C₄-C₆ oléfinique

On utilise un catalyseur préparé de façon identique au catalyseur A décrit ci-dessus pour alkyler l'isobutane par une coupe C₄-C₆ oléfinique.

La coupe utilisée a la composition pondérale suivante :

| | |
|---|---|
| 2-butène | 10 % |
| 1-pentène | 35 % |
| hexènes | 55 % |

La coupe hexène contient 60 % de méthyl-2-pentène-2 et 40 % d'hexène-2.

On opère comme décrit précédemment pour la réaction d'alkylation et on alkyle 90 ml d'isobutane par 7,4 ml de la charge oléfinique décrite ci-dessus pendant 4 heures et 30 minutes à la température de -15° C. L'alkylat est recueilli et analysé par chromatographie en phase vapeur. La conversion des oléfines est totale. La composition pondérale de l'alkylat obtenu est donnée dans le tableau 2 ci-après.

**Tableau 2**

| | |
|---|---|
| iC₅ | 3 |
| C₆ | 18 |
| C₇ | 3 |
| C₈ | 25 |
| C₉ | 25 |
| C₉⁺ | 26 |

La fraction C₆ contient 73 % de méthylpentanes, la coupe C₈ contient 92 % poids de triméthylpentanes et la fraction C₉ contient 89 % de triméthylhexanes.

### Exemple 2 (selon l'invention). CATALYSEUR ACIDE SULFURIQUE SUR SILICE.

### Préparation du catalyseur B

On active 15 g de silice macroporeuse de surface spécifique égale à 27 m²/g et de volume poreux total égal à 0,78 cm³/g, par calcination sous air pendant 2 heures à 500°C. La silice ainsi activée est conservée sous argon. On procède alors à une imprégnation à sec de 13,7 g de ladite silice par 18,96 g d'une solution d'acide sulfurique à 96 % en poids. Le solide ainsi obtenu, appelé catalyseur B, contient 18,96 g d'acide sulfurique et 13,7 g de silice, et est conservé sous argon à -20 °C.

### Alkylation de l'isobutane par le butène-1

On introduit 32 g du catalyseur B préparé selon la méthode décrite ci-dessus dans un réacteur en verre du type Fischer & Porter d'un volume de 360 ml préalablement purgé sous débit d'argon. Le réacteur contenant le catalyseur B est alors fermé, mis sous vide primaire, puis il est refroidi à la température de -20 °C.

57 cm³ d'isobutane sont alors ajouté dans le réacteur contenant le catalyseur sous agitation, ledit réacteur étant immergé dans un bain froid à -20 °C. On laisse sous agitation le système catalyseur B + isobutane pendant une durée de 30 minutes afin d'homogénéiser la température.

On ajoute régulièrement 1,70 cm³ de butène-1 par heure pendant une durée totale de 6 heures, la température du réacteur est maintenu à -7 °C pendant toute la durée de l'injection.

Après réaction, la phase hydrocarbure est soutirée du réacteur, puis l'isobutane est évaporé lentement et on recueille l'alkylat qui est analysé par chromatographie en phase vapeur. Sa composition pondérale est donnée dans le tableau 3 ci-après. La conversion de l'oléfine est de 100 %.

**Tableau 3**

| | |
|---|---|
| iC₅ | 1,26 |
| C₆ | 3,16 |
| C₇ | 2,62 |
| C₈ | 83 |
| C₉ | 1,70 |
| C₉⁺ | 8,26 |

La fraction C₈ contient 89,7 % poids de triméthylpentanes et la fraction C₉ contient 92,2 % de triméthylhexanes.

### Exemple 3 (selon l'invention). CATALYSEUR ACIDE SULFURIQUE + ANHYDRIDE SULFURIQUE SUR SILICE.

### Préparation du catalyseur C

On active 14 g de silice macroporeuse, de surface spécifique égale à 27 m²/g et de volume poreux égal à 1 cm³/g, par calcination sous air pendant 4 heures à 500°C. Le solide ainsi activé est conserve sous argon. On procède alors à une imprégnation à sec de 10 g du solide calciné par 7 cm³ du mélange constitué de 80% poids d'acide sulfurique (à 99,99%) et 20% poids d'anhydride sulfurique. Le catalyseur ainsi obtenu, catalyseur C, contient 13,5 g d'oléum et 10 g de silice et est conservé sous atmosphère d'argon à -18 °C.

### Alkylation de l'isobutane par le butène-1 avec le catalyseur C

On introduit 20 g du catalyseur C préparé selon la méthode décrite dans l'exemple 3 dans un réacteur en verre du type Fischer & Porter d'un volume de 360 cm³ préalablement purgé sous débit d'argon. Le réacteur contenant le catalyseur est alors fermé puis mis sous vide primaire, puis il est refroidi à la température de -20°C.

72 cm³ d'isobutane sont alors ajoutés dans le réacteur contenant le catalyseur sous agitation, ledit réacteur étant immergé dans un bain froid à -20 °C. On laisse sous agitation le système catalyseur + isobutane pendant une durée de 30 minutes afin d'homogénéiser la température.

On ajoute régulièrement 50 cm³ d'un mélange constitué de 24% en volume de butène-1 et de 76% en volume d'isobutane, pendant une durée totale de 10 heures, la température du réacteur étant maintenue à -15 °C pendant toute la durée de l'injection.

Après réaction, la phase hydrocarbure est soutirée du réacteur, l'isobutane est évaporé lentement. On recueille l'alkylat qui est analysé par chromatographie en phase vapeur ; sa composition pondérale est donnée ci-dessous (tableau 4).

**Tableau 4**

| | |
|---|---|
| iC₅ | 1,5 |
| C₆ | 1,1 |
| C₇ | 1,9 |
| C₈ | 90 |
| C₉ | 1,2 |
| C₉⁺ | 4,3 |

La conversion de l'oléfine est de 98 %. Le rendement de l'alkylation est de 200% par rapport à l'oléfine tranformée. La fraction C₈ contient 89% poids de triméthylpentanes.

### Exemple 4 (selon l'invention). PREPARATION D'UN CATALYSEUR ACIDE SULFURIQUE + ANHYDRIDE SULFURIQUE + ACIDE BORIQUE SUR SILICE.

### Préparation du catalyseur D

Pour préparer le catalyseur D, on utilise 13 g de la même silice macroporeuse que celle utilisée pour la préparation du catalyseur C, les conditions de calcination sont identiques. On prépare un mélange constitué d'oléum et d'acide borique : pour cela on utilise 7 cm³ du même mélange que celui utilisé pour l'obtention du catalyseur C, auxquels on ajoute 0,81 g d'acide borique anhydre. On obtient alors 14,31 g d'un mélange d'acide sulfurique (75,47% poids), d'anhydride sulfurique (18,86% poids) et d'acide borique (5,67% poids).

On procède alors à une imprégnation à sec de 11 g de la silice calcinée par la totalité du mélange décrit ci-avant. Le catalyseur ainsi obtenu, catalyseur D, contient 14,31 g de phase acide et 11 g de silice, et est conservé sous atmosphère d'argon à -18 °C.

### Alkylation de l'isobutane par le butène-1 avec le catalyseur D

On répète le test catalytique d'alkylation de l'isobutane par le butène-1 dans les mêmes conditions expérimentales que celles décrites dans l'exemple 3. Les résultats sont rassemblés dans le tableau 5 ci-dessous.

**Tableau 5**

| | |
|---|---|
| iC₅ | 0,8 |
| C₆ | 0,5 |
| C₇ | 1,1 |
| C₈ | 94,6 |
| C₉ | 0,9 |
| C₉⁺ | 2,1 |

Le rendement de l'alkylation est de 201% par rapport à l'oléfine tranformée. La fraction C₈ contient 92% poids de triméthylpentanes.

Ce tableau met en évidence l'intérêt qu'il y a à ajouter au mélange acide sulfurique - anhydride sulfurique de l'acide borique, ce qui constitue l'un des modes préféré de la réalisation de l'invention.

## Revendications

1. Utilisation d'un catalyseur comprenant de la silice et une phase acide à l'état solide comprenant de l'acide sulfurique de concentration comprise entre 5 et 100 % poids, la silice ayant été imprégnée par ladite phase acide et ayant une surface spécifique comprise entre 0,01 et 1500 m²/g et un volume poreux total compris entre 0,005 et 1,5 cm³/g, dans un procédé d'alkylation catalytique d'au moins un élément choisi dans le groupe formé par l'isobutane et l'isopentane par au moins un élément choisi dans le groupe formé par les oléfines comprenant de 3 à 6 atomes de carbone par molécule.

2. Utilisation d'un catalyseur défini dans la revendication 1 tel que la silice, avant son imprégnation par la phase acide, comprend au plus 5 % d'impuretés.

3. Utilisation d'un catalyseur défini dans l'une des revendications 1 ou 2 tel que la phase acide comprend en outre au moins un additif.

4. Utilisation d'un catalyseur défini dans la revendication 3 tel que l'additif est choisi dans le groupe formé par BO₃H, FSO₃H, CF₃SO₃H, SbF₅, CF₃COOH, SO₃, H₃PO₄ et BF₄H.

5. Utilisation d'un catalyseur défini dans l'une des revendications 3 ou 4, tel que l'additif est l'anhydride sulfurique SO₃, la teneur pondérale en anhydride sulfurique dans ladite phase acide étant comprise entre 0,01 et 60 %.

6. Utilisation d'un catalyseur défini selon l'une des revendications 3 à 5, tel que la phase acide contient de l'acide sulfurique, de l'anhydride sulfurique et de l'acide borique, la teneur pondérale en anhydride sulfurique dans ladite phase acide étant comprise entre 0,01 et 60 % et la teneur pondérale en acide borique dans le mélange comprenant l'acide sulfurique et l'anhydride sulfurique étant comprise entre 0,01 et 50 %.

7. Utilisation d'un catalyseur selon l'une des revendications 1 à 6 préparé par un procédé tel que la silice est calcinée puis imprégnée par de ladite phase acide.

8. Utilisation selon l'une des revendications 1 à 7 dans laquelle la température de la réaction est inférieure à 0° C.

9. Utilisation d'un catalyseur selon l'une des revendications 1 à 8 pour un procédé d'alkylation catalytique d'au moins un élément choisi dans le groupe formé par l'isopentane et l'isobutane par au moins un élément choisi dans le groupe formé par les oléfines comprenant de 3 à 6 atomes de carbone par molécule, ledit procédé étant tel que le catalyseur est mis en oeuvre en lit mobile.

10. Utilisation d'un catalyseur selon l'une des revendications 1 à 8 pour un procédé d'alkylation catalytique d'au moins un élément choisi dans le groupe formé par l'isopentane et l'isobutane par au moins un élément choisi dans le groupe formé par les oléfines comprenant de 3 à 6 atomes de carbone par molécule, ledit procédé étant tel que le catalyseur est mis en oeuvre en lit fluide.

11. Utilisation d'un catalyseur selon l'une des revendications 1 à 8 pour un procédé d'alkylation catalytique d'au moins un élément choisi dans le groupe formé par l'isopentane et l'isobutane par au moins un élément choisi dans le groupe formé par les oléfines comprenant de 3 à 6 atomes de carbone par molécule, et ledit procédé étant tel que le catalyseur est mis en oeuvre en suspension dans la phase liquide des réactifs soumise à une agitation efficace.

## Claims

1. Use of a catalyst comprising silica and an acid phase in the solid state comprising sulphuric acid with a concentration between 5 and 100 % by weight, the silica having been impregnated by said acid phase and having a specific surface between 0.01 and 1500 m²/g and a total pore volume of between 0.005 and 1.5 cm³/g.

2. Use of a catalyst according to claim 1, wherein the silica, prior to its impregnation by the acid phase, contains at the most 5 % impurities.

3. Use of a catalyst according to one of the claims 1 or 2, wherein the acid phase also contains at least one additive.

4. Use of a catalyst according to claim 3, wherein the additive is chosen from within the group formed by BO₃H, FSO₃H, CF₃SO₃H, SbF₅, CF₃COOH, SO₃, H₃PO₄ and BF₄H.

5. Use of a catalyst according to one of the claims 3 or 4, wherein the additive is sulphur trioxide SO₃, the sulphur trioxide weight content in said acid phase being between 0.01 and 60 %.

6. Use of a catalyst according to any one of the claims 3 to 5, wherein the acid phase contains sulphuric acid, sulphur trioxide and boric acid, the sulphur trioxide weight content in said acid phase being between 0.01 and 60 % and the boric acid weight content in the mixture comprising sulphuric acid and sulphur trioxide being between 0.01 and 50 %.

7. Use of a catalyst according to any one of the claims 1 to 6, prepared by a process wherein the silica is calcined and then impregnated by said acid phase.

8. Use of the catalyst according to any one of the claims 1 to 7, wherein the reaction temperature is below 0° C.

9. Use of a catalyst according to one of the claims 1 to 8 in a process for the catalytic alkylation of at least one element chosen from within the group formed by isobutane and isopentane by at least one element chosen from within the group formed by olefins having 3 to 6 carbon atoms per molecule, such process being such that the catalyst is used in a moving bed.

10. Use of a catalyst according to one of the claims 1 to 8 in a process for the catalytic alkylation of at least one element chosen from within the group formed by isobutane and isopentane by at least one element chosen from within the group formed by olefins having 3 to 6 carbon atoms per molecule, such process being such that the catalyst is used in a fluid bed.

11. Use of a catalyst according to one of the claims 1 to 8 in a process for the catalytic alkylation of at least one element chosen from within the group formed by isobutane and isopentane by at least one element chosen from within the group formed by olefins having 3 to 6 carbon atoms per molecule, such process being such that the catalyst is suspended in the liquid phase of the reagents subject to an effective stirring.

## Patentansprüche

1. Verwendung eines Katalysators, der Siliciumdioxid und eine saure Phase im Festzustand umfaßt, die Schwefelsäure einer Konzentration zwischen 5 und 100 Gew.% umfaßt, wobei das Siliciumdioxid durch diese saure Phase imprägniert wurde und eine spezifische Oberfläche, die zwischen 0,01 und 1.500 m²/g enthalten ist, und ein Gesamtporenvolumen, das zwischen 0,005 und 1,5 cm³/g enthalten ist, aufweist, in einem Verfahren zur katalytischen Alkylierung wenigstens eines Grundstoffes, der aus der Gruppe ausgewählt ist, die durch Isobutan und Isopentan gebildet ist, durch wenigstens einen Grundstoff der aus der Gruppe ausgewählt ist, die durch die Olefine, welche 3 bis 6 Kohlenstoffatome pro Molekül enthalten, gebildet ist.

2. Verwendung eines Katalysators gemäß Anspruch 1, so daß das Siliciumdioxid vor seiner Imprägnierung durch die saure Phase höchstens 5 % Verunreinigungen umfaßt.

3. Verwendung eines Katalysators gemäß einem der Ansprüche 1 oder 2, so daß die saure Phase des weiteren wenigstens ein Additiv umfaßt.

4. Verwendung eines Katalysators gemäß Anspruch 3, so daß das Additiv aus der Gruppe ausgewählt ist, die durch BO₃H, FSO₃H, CF₃SO₃H, SbF₅, CF₃COOH, SO₃, H₃PO₄ und BF₄H gebildet ist.

5. Verwendung eines Katalysators gemäß einem der Ansprüche 3 oder 4, so daß das Additiv Schwefelsäureanhydrid SO₃ ist, wobei der Gewichtsgehalt an Schwefelsäureanhydrid in dieser sauren Phase zwischen 0,01 und 60 % enthalten ist.

6. Verwendung eines Katalysators gemäß einem der Ansprüche 3 bis 5, so daß die saure Phase Schwefelsäure, Schwefelsäureanhydrid und Borsäure enthält, wobei der Gewichtsgehalt an Schwefelsäureanhydrid in der sauren Phase zwischen 0,01 und 60 % enthalten ist und wobei der Gewichtsgehalt an Borsäure in der die Schwefelsäure und das Schwefelsäureanhydrid enthaltenden Mischung zwischen 0,01 und 50 % enthalten ist.

7. Verwendung eines Katalysators gemäß einem der Ansprüche 1 bis 6, hergestellt durch ein Verfahren, so daß das Siliciumdioxid kalziniert, dann durch die saure Phase imprägniert, wird.

8. Verwendung gemäß einem der Ansprüche 1 bis 7, worin die Temperatur der Reaktion unter 0°C liegt.

9. Verwendung eines Katalysators gemäß einem der Ansprüche 1 bis 8, für ein Verfahren zur katalytischen Alkylierung wenigstens eines Grundstoffes, der aus der Gruppe ausgewählt ist, die durch Isopentan und Isobutan gebildet ist, durch wenigstens einen Grundstoff, der aus der Gruppe ausgewählt ist, die durch die Olefine, welche 3 bis 6 Kohlenstoffatome pro Molekül enthalten, gebildet ist, wobei das Verfahren derart ist, daß der Katalysator im Wirbelbett eingesetzt wird.

10. Verwendung eines Katalysators gemäß einem der Ansprüche 1 bis 8, für ein Verfahren zur katalytischen Alkylierung wenigstens eines Grundstoffes, der aus der Gruppe ausgewählt ist, die durch Isopentan und Isobutan gebildet ist, durch wenigstens einen Grundstoff, der aus der Gruppe ausgewählt ist, die durch die Olefine, welche 3 bis 6 Kohlenstoffatome pro Molekül enthalten, gebildet ist, wobei das Verfahren derart ist, daß der Katalysator im Fließbett eingesetzt wird.

11. Verwendung eines Katalysators gemäß einem der Ansprüche 1 bis 8, für ein Verfahren zur katalytischen Alkylierung wenigstens eines Grundstoffes, der aus der Gruppe ausgewählt ist, die durch Isopentan und Isobutan gebildet ist, durch wenigstens einen Grundstoff, der aus der Gruppe ausgewählt ist, die durch die Olefine, welche 3 bis 6 Kohlenstoffatome pro Molekül enthalten, gebildet ist, wobei das Verfahren derart ist, daß der Katalysator in Suspension in der flüssigen Phase der einem wirksamen Rühren unterworfenen Reagenzien eingesetzt wird.
